Europäisches Patentamt

European Patent Office

Office européen des brevets

(11) Veröffentlichungsnummer: **0 212 596**
**A2**

(12) # EUROPÄISCHE PATENTANMELDUNG

(21) Anmeldenummer: **86111355.3**

(22) Anmeldetag: **16.08.86**

(51) Int. Cl.⁴: **A 61 K 47/00,** A 61 K 9/08,
A 61 K 31/557
// C07C177/00

(30) Priorität: **29.08.85 DE 3530821**

(43) Veröffentlichungstag der Anmeldung: **04.03.87**
**Patentblatt 87/10**

(84) Benannte Vertragsstaaten: **AT BE CH DE FR GB IT LI NL
SE**

(71) Anmelder: **Merck Patent Gesellschaft mit beschränkter
Haftung, Frankfurter Strasse 250, D-6100 Darmstadt
(DE)**

(72) Erfinder: **Oelrich, Eckhard, Dr., Auf der Schmelz 19,
D-6101 Rossdorf (DE)**
Erfinder: **Starz, Carola, Beckstrasse 4,
D-6100 Darmstadt (DE)**
Erfinder: **Wotschokowsky, Manfred, Dr.,
Langgässerweg 52, D-6100 Darmstadt (DE)**

(54) **Luprostiolhaltige Lösung.**

(57) Die Erfindung betrifft eine neue Lösung, enthaltend
Luprostiol und 1,2-Propandiol, dadurch gekennzeichnet,
dass sie Luprostiol in Form seines Natriumsalzes in einem
Gemisch aus 50–90 Gewichtsprozent 1,2-Propandiol und
50–10 Gewichtsprozent Wasser enthält und einen pH-Wert
zwischen etwa 6 und 8 besitzt.

0 212 596

**Merck Patent Gesellschaft**
**mit beschränkter Haftung**

**6100  D a r m s t a d t**

Luprostiolhaltige Lösung

**Gegenstand der Erfindung ist eine neue Lösung, enthaltend Luprostiol und 1,2-Propandiol, dadurch gekennzeichnet, daß sie Luprostiol in Form seines Natriumsalzes in einem Gemisch aus 50-90 Gewichtsprozent 1,2-Propandiol und 50-10 Gewichtsprozent Wasser enthält und einen pH-Wert zwischen etwa 6 und 8 besitzt.**

**Lösungen von Luprostiol (= 7α-(2-(3-(3-Chlorphenoxy)-2-hydroxy-propylthio)-3α,5α-dihydroxy-cyclopentyl)-5-heptensäure) in 1,2-Propandiol sind aus der CA-PS 1 142 434 bekannt. Sie werden insbesondere als Injektionslösungen in der Human- und vor allem in der Veterinärmedizin verwendet, dort vorzugsweise zur Luteolyse und zur Brunstsynchronisation oder zur Geburtseinleitung, z. B. bei Pferden, Rindern, Schweinen, Schafen und Ziegen.**

**Bei der Anwendung der bekannten Lösungen zeigten sich Nachteile. Insbesondere stört die hohe Viskosität, die das Aufziehen und Spritzen erheblich erschwert, insbesondere bei niedrigen Temperaturen wie sie bei der vorwiegenden praktischen Anwendung im Winterhalbjahr herrschen. Weiterhin sind Lösungen der freien Säure Luprostiol nicht unbeschränkt stabil, da durch saure Autokatalyse Ester aus der Säure und dem Lösungsmittel entstehen.**

PAT LOG 5/1 260885

Der Erfindung lag daher die Aufgabe zugrunde, diese Nachteile der bekannten Lösungen zu vermeiden oder mindestens zu mildern, insbesondere eine chemisch und galenisch stabile niedrig-viskose Lösung aufzufinden, die eine ausreichend hohe Wirkstoffkonzentration zuläßt und lokal gut verträglich ist.

Diese Aufgabe wurde gelöst durch die Bereitstellung der beanspruchten Lösung. Diese ist chemisch und galenisch stabil, selbstkonservierend und wesentlich niedrigerviskos als die bekannten Lösungen, so daß sie sich hinsichtlich der Viskosität bei allen in der Praxis üblichen Temperaturen ohne Probleme handhaben läßt. Überdies ist die Lösung lokal besser verträglich als die herkömmliche.

Die erfindungsgemäße Lösung enthält zweckmäßig 0,1 bis 100, vorzugsweise 1 bis 10, insbesondere 3 bis 8 mg Luprostiol-Natriumsalz/ml. Das Lösungsmittelgemisch enthält 50 bis 90, vorzugsweise 60 bis 80, insbesondere 65 bis 75 Gewichtsprozent 1,2-Propandiol und dementsprechend 50 bis 10, vorzugsweise 40 bis 20, insbesondere 35 bis 25 Gewichtsprozent Wasser. Der pH-Wert der Lösung liegt zwischen etwa 6 und 8, vorzugsweise zwischen 6,5 und 7,5, insbesondere zwischen 6,8 und 7,2.

Gegenstand der Erfindung ist ferner ein Verfahren zur Herstellung einer Lösung, enthaltend Luprostiol und 1,2-Propandiol, dadurch gekennzeichnet, daß man Luprostiol oder dessen Natriumsalz in 1,2-Propandiol löst, die Lösung mit Wasser und gegebenenfalls weiterem 1,2-Propandiol auf eine Konzentration bringt, bei der das Gemisch 50-90 Gewichtsprozent 1,2-Propandiol und 50-10 Gewichtsprozent Wasser enthält, und gegebenenfalls durch Zugabe einer basischen Natriumverbindung auf einen pH-Wert zwischen etwa 6 und 8 einstellt.

Im einzelnen stellt man zunächst zweckmäßig eine etwa 12- bis 13 %ige Lösung von Luprostiol in 1,2-Propandiol her, versetzt mit weiterem 1,2-Propandiol und mit der berechneten Menge Wasser, stellt mit Natronlauge auf den gewünschten pH-Wert ein und füllt mit 1,2-Propandiol auf.

An Stelle von Natronlauge (Natriumhydroxid-Lösung) eignen sich als basische Natriumverbindungen z. B. auch Natriumcarbonat oder -bicarbonat, zweckmäßig in Form ihrer wässerigen Lösungen.

Die bisher beschriebenen Operationen werden zweckmäßig bei Temperaturen zwischen 10 und 80°, vorzugsweise zwischen 20 und 30° durchgeführt, vorteilhaft unter Rühren.

Die so erhaltene Lösung wird zweckmäßig unter aseptischen Bedingungen und, falls erwünscht, unter einem Inertgas wie Stickstoff durch ein Membranfilter mit einer Porenweite von 0,1 bis 0,4, vorzugsweise 0,2 μm, steril filtriert. Die niedrige Viskosität der Lösung bewirkt, daß sie schneller filtriert werden kann als die bekannte Lösung höherer Viskosität. Damit sind verbunden ein Zeitgewinn bei der Herstellung, eine kürzere und damit geringere Belastung des Wirkstoffs sowie eine kürzere Zeit für eine mögliche Aufnahme von Verunreinigungen.

Die erhaltene Lösung kann in Ampullen oder Injektionsgläser abgefüllt werden, zweckmäßig unter aseptischen Bedingungen und Lichtschutz. Die Ansatzgefäße können evakuiert und mit einem Inertgas wie Stickstoff gefüllt werden. Jede Ampulle bzw. jedes Injektionsglas kann z. B. zwischen 0,1 und 1000, vorzugsweise zwischen 0,5 und 100 mg Wirkstoff enthalten.

PAT LOG 5/1 260885

Die neue Lösung kann in der Human- oder, vorzugsweise, in der Veterinärmedizin verwendet werden. Sie wird vorzugsweise parenteral als Injektionslösung, insbesondere intramuskulär oder intravenös appliziert, kann aber auch oral, z. B. in Form von Tropfen verabreicht werden. Sie wird vorzugsweise in einer Dosierung von 0,001 bis 1, insbesondere 0,005 bis 0,1 mg/kg Körpergewicht verabreicht; die Dosierung ist abhängig von der behandelten Spezies, der Applikationsform und dem Behandlungszweck, sie kann daher die oben angegebenen Werte auch unter- bzw. überschreiten.

Will man beispielsweise die Östrus synchronisierende Wirkung des Luprostiols ausnützen, so ist es besonders vorteilhaft, z. B. Rindern (Kühen bzw. Färsen) 0,1 mg bis 20 mg, vorzugsweise 0,5 mg bis 15 mg, insbesondere 1,5 mg bis 10 mg des Wirkstoffs intramuskulär zu injizieren. Es ist günstig, die wirksame Dosis durch einmalige Injektion zwischen dem 7. Tag und dem 12. Tag des Zyklus zu verabreichen, man kann aber auch mehrmals und gegebenenfalls über mehrere Tage verteilt Teildosen injizieren. Auch bei anderen Nutztieren, beispielsweise bei Hunden, Pferden, Schafen und Schweinen kann der Östrus durch Verabreichung von Luprostiol synchronisiert werden. Die wirksame Dosis variiert dabei in Abhängigkeit vom mittleren Körpergewicht der behandelten Spezies und kann unschwer vom Fachmann mit Hilfe der oben für Rinder angegebenen Richtwerte ermittelt werden.

Beispiel 1

60,19 g einer 12,46-proz. Lösung von Luprostiol in 1,2-Propandiol werden nacheinander mit 600 g 1,2-Propandiol und 298,8 g Wasser für Injektionszwecke versetzt und gerührt, bis eine klare Lösung vorliegt. Der pH-Wert dieser Lösung wird mit 16,7 ml 1 M Natronlauge auf 7,0 eingestellt. Die Lösung wird mit 1,2-Propandiol bis zum Endgewicht von 1048 g = 1000 ml aufgefüllt, unter aseptischen Bedingungen durch ein Membranfilter steril filtriert und in 10 ml-Injektionsgläser gefüllt. 1 ml dieser Injektionslösung enthält 7,5 mg Luprostiol. Viskosität: 14 mPas bei 20°, 44 mPas bei 0°. (Eine Lösung von 7,5 mg/ml Luprostiol in reinem 1,2-Propandiol hat dagegen folgende Viskositäten: 61 mPas bei 20°, 257 mPas bei 0°).

Beispiel 2

23,47 g einer 12,78-proz. Lösung von Luprostiol in 1,2-Propandiol werden analog Beispiel 1 mit 650 g 1,2-Propandiol, 313,7 g Wasser für Injektionszwecke und 6,7 ml 1 M Natronlauge versetzt und danach mit 1,2-Propandiol zum Endgewicht von 1045 g = 1000 ml aufgefüllt. Die Lösung vom pH-Wert 7,0 wird steril filtriert und in 10-ml-Injektionsgläser gefüllt. 1 ml dieser Injektionslösung enthält 3,0 mg Luprostiol.

Beispiel 3

Analog Beispiel 1 erhält man aus 55,8 g einer 13,5-proz. Lösung von Luprostiol in 1,2-Propandiol, 404,5 g Wasser für Injektionszwecke, 570,6 g 1,2-Propandiol und 16,1 g 1 M Natronlauge 1000 ml einer Injektionslösung, die 7,5 mg/ml Luprostiol enthält. Viskosität: 10,1 mPas bei 20°, 29,4 mPas bei 0°.

Merck Patent Gesellschaft
mit beschränkter Haftung

6100  D a r m s t a d t

## Patentansprüche

1.  Lösung, enthaltend Luprostiol und 1,2-Propandiol, dadurch gekennzeichnet, daß sie Luprostiol in Form seines Natriumsalzes in einem Gemisch aus 50-90 Gewichtsprozent 1,2-Propandiol und 50-10 Gewichtsprozent Wasser enthält und einen pH-Wert zwischen etwa 6 und 8 besitzt.

2.  Verfahren zur Herstellung einer Lösung, enthaltend Luprostiol und 1,2-Propandiol, dadurch gekennzeichnet, daß man Luprostiol oder dessen Natriumsalz in 1,2-Propandiol löst, die Lösung mit Wasser und gegebenenfalls weiterem 1,2-Propandiol auf eine Konzentration bringt, bei der das Gemisch 50-90 Gewichtsprozent 1,2-Propandiol und 50-10 Gewichtsprozent Wasser enthält, und gegebenenfalls durch Zugabe einer basischen Natriumverbindung auf einen pH-Wert zwischen etwa 6 und 8 einstellt.

3.  Verwendung einer Lösung nach Anspruch 1 zur Luteolyse und/oder zur Brunstsynchronisation oder zur Geburtseinleitung.

PAT LOG 13 220885